(19) Europäisches Patentamt
European Patent Office
Office européen des brevets

(11) **EP 0 457 853 B1**

(12) **EUROPEAN PATENT SPECIFICATION**

(45) Date of publication and mention
of the grant of the patent:
**06.08.1997 Bulletin 1997/32**

(21) Application number: **90906587.2**

(22) Date of filing: **09.04.1990**

(51) Int Cl.[6]: **C07C 6/12**

(86) International application number:
**PCT/US90/01895**

(87) International publication number:
**WO 91/08998 (27.06.1991 Gazette 1991/14)**

(54) **A PROCESS FOR THE CATALYTIC CONVERSION OF A C9+ AROMATICS FEED**

VERFAHREN ZUR KATALYTISCHEN KONVERSION EINER C9+ -AROMATISCHEN CHARGE

PROCEDE DE CONVERSION CATALYTIQUE D'UNE CHARGE DE COMPOSES C9+ AROMATIQUES

(84) Designated Contracting States:
**AT BE CH DE DK ES FR GB IT LI LU NL SE**

(30) Priority: **13.12.1989 US 449169**
**24.01.1990 US 469645**

(43) Date of publication of application:
**27.11.1991 Bulletin 1991/48**

(73) Proprietor: **MOBIL OIL CORPORATION**
**New York New York 10017 (US)**

(72) Inventors:
• **ABSIL, Robert, Peter, Leonard**
**Mantua, NJ 08051 (US)**
• **CHANG, Clarence, Dayton**
**Princeton, NJ 08540 (US)**
• **HAN, Scott**
**Lawrenceville, NJ 08648 (US)**
• **MARLER, David, Owen**
**Deptford, NJ 08096 (US)**

• **SHIHABI, David, Said**
**Pennington, NJ 08534 (US)**

(74) Representative: **Colmer, Stephen Gary et al**
**Mobil Services Company Limited,**
**Office of Legal Counsel,**
**European IP Group,**
**Mobil Court,**
**3 Clements Inn**
**London WC2A 2EB (GB)**

(56) References cited:
| | |
|---|---|
| DE-A- 2 714 239 | US-A- 4 046 827 |
| US-A- 4 418 235 | US-A- 4 575 573 |
| US-A- 4 577 048 | US-A- 4 599 470 |
| US-A- 4 891 458 | |

Remarks:
The file contains technical information submitted after the application was filed and not included in this specification

## Description

This invention relates to a process for the catalytic conversion of a $C_9+$ aromatic feedstock.

Zeolitic materials, both natural and synthetic, have been demonstrated in tne past to have catalytic properties for various types of hydrocarbon conversion. Certain zeolitic materials are ordered, porous crystalline aluminosilicates having a definite crystalline structure as determined by X-ray diffraction, within which there are a large number of smaller cavities which may be interconnected by a number of still smaller channels or pores. These cavities and pores are uniform in size within a specific zeolitic material. Since the dimensions of these pores are such as to accept for adsorption molecules of certain dimensions while rejecting those of larger dimensions, these materials have come to be known as "molecular sieves" and are utilized in a variety of ways to take advantage of these properties. Such molecular sieves, both natural and synthetic, include a wide variety of positive ion-containing crystalline silicates. These silicates can be described as a rigid three-dimensional framework of $SiO_4$ and Periodic Table Group IIIA element oxide, e.g., $AlO_4$, in which the tetrahedra are cross-linked by the sharing of oxygen atoms whereby the ratio of the total Group IIIA element, e.g., aluminum, and silicon atoms to oxygen atoms is 1:2. The electrovalence of the tetrahedra containing the Group IIIA element, e.g., aluminum, is balanced by the inclusion in the crystal of a cation, e.g., an alkali metal or an alkaline earth metal cation. This can be expressed wherein the ratio of the Group IIA element, e.g., aluminum, to the number of various cations, such as Ca/2, Sr/2, Na, K or Li, is equal to unity. One type of cation may be exchanged either entirely or partially with another type of cation utilizing ion exchange techniques in a conventional manner. By means of such cation exchange, it has been possible to vary the properties of a given silicate by suitable selection of the cation.

Prior art techniques have resulted in the formation of a great variety of synthetic zeolites. Many of these zeolites have come to be designated by letter or other convenient symbols, as illustrated by zeolite Z (U.S. Patent No. 2,882,243), zeolite X (U.S. Patent No. 2,882,244), zeolite Y (U.S. Patent No. 3,130,007), zeolite ZK-5 (U.S. Patent No. 3,247,195), zeolite ZK-4 (U.S. Patent No. 3,314,752), zeolite ZSM-5 (U.S. Patent No. 3,702,886), zeolite ZSM-11 (U. S. Patent No. 3,709,979), zeolite ZSM-12 (U.S. Patent No. 3,832,449), zeolite ZSM-20 (U.S. Patent No. 3,972,983), zeolite ZSM-35 (U.S. Patent No. 4,016,245), and zeolite ZSM-23 (U.S. Patent No. 4,076,842).

The $SiO_2/Al_2O_3$ ratio of a given zeolite is often variable. For example, zeolite X can be synthesized with $SiO_2/Al_2O_3$ ratios of from 2 to 3; zeolite Y, from 3 to 6. In some zeolites, the upper limit of the $SiO_2/Al_2O_3$ ratio is unbounded. ZSM-5 is one such example wherein the $SiO_2/Al_2O_3$ ratio is at least 5 and up to the limits of present analytical measurement techniques. U.S. Patent No. 3,941,871 (Re. 29,948) discloses a porous crystalline silicate made from a reaction mixture containing no deliberately added alumina in the recipe and exhibiting the X-ray diffraction pattern characteristic of ZSM-5. U.S. Patent Nos. 4,061,724, 4,073,865 and 4,104,294 describe crystalline silicates of varying alumina and metal content.

U.S. Patent No. 4,380,685 discloses the para-selective alkylation, transalkylation or disproportionation of a substituted aromatic compound to provide a mixture of dialkylbenzene compounds employing as catalyst a zeolite characterized by a Constraint Index of 1 to 12 and a silica/alumina mole ratio of at least 12/1, the catalyst having incorporated thereon various metals and phosphorus. Other patents covering alkylation and transalkylation processes include U.S. Patent Nos. 4,127,616; 4,361,713, 4,365,104; 4,367,359; 4,370,508; and, 4,384,155. Conversion of toluene to para-xylene is disclosed in U.S. Patent Nos. 3,965,207; 3,965,208; 3,965,209; 4,001,346; 3,002,698; 4,067,920; 4,100,215; and, 4,152,364. Alkylation of aromatics with olefins is disclosed in, for example, U.S. Patent Nos. 3,962,364 and 4,016,218.

U.S. Patent No. 3,551,509 and U.S. Patent Re. 27,639 disclose transalkylation between trimethylbenzenes and toluene to yield xylenes and benzene in the presence of a crystalline aluminosilicate catalyst having large pore openings of 8 to 15 Angstrom units and preferably containing Group VIII metals, hydrogen and rare earth cations.

U.S. Patent Nos. 3,126,422; 3,413,374; 3,598,878; 3,598,879; and, 3,607,961 describe the vapor-phase disproportionation of toluene over various catalysts. U.S. Patent No. 4,117,026 discloses disproportionation of toluene over a catalyst comprising a zeolite having a silica/alumina mole ratio of at least 12, a Constraint Index of 1 to 12 and a specified sorption capacity for xylenes.

U.S. Patent Re. 31,781 (of original U.S. Patent Nos. 4,100,214) discloses the use of from 3 to 30 combined weight percent of toluene and $C_9+$ recycle material as diluents with a monocyclic alkyl aromatic hydrocarbon feed selected from the xylenes, mesitylene, durene, hemimellitene, pseudocumene, prehnitene, isodurene and 1,3,5-triethylbenzene for the vapor-phase isomerization of said feed employing as catalyst, a zeolite having a Constraint Index of 1 to 12, e. g., ZSM-5, ZSM-11, ZSM-12, ZSM-35 and ZSM-38.

The present invention resides in a process for converting a feedstock containing at least one monoonuclear aromatic compound having at least 9 carbon atoms to an aromatic product containing 6-8. carbon atorns which comprises contacting the feedstock in the presence or absence of hydrogen with a zeolite catalyst having an X-ray diffraction pattern including the lines set out in Table I below.

The $C_9+$ aromatics feed employed in the process of this invention comprises one or more mononuclear aromatic compounds containing at least 9 carbons such as, e.g., trimethylbenzenes, dimethylethylbenzenes and diethylbenzenes. Specific $C_9+$ aromatic compounds include mesitylene (1,3,5-trimethylbenzene), durene (1,2,4,5-trimethylbenzene) such as is obtained as a by-product of the conversion of $C_1$-$C_4$ oxygenates to gasoline, hemimellitene (1,2,3-trimethylbenzene), pseudocumene (1,2,4-trimethylbenzene), prehnitene (1,2,3,4-tetramethylbenzene), isodurene (1,2,3,5-tetramethylbenzene), and mesitylene (1,3,5-triethylbenzene). Using such mononuclear aromatic feeds, it is found that the process of the invention can generate products having higher xylene/benzene mole ratios than those obtainable with zeolite catalysts having a Constraint Index of greater than 3, e.g., ZSM-5 which possesses a Constraint Index 6-8.3 (when measured at 371°C - 316°C). The product xylene/benzene mole ratios herein will generally be greater than 0.80, usually greater than 0.90, and often greater than 1.

The feedstock employed in the present process conveniently contains benzene or more preferably toluene in addition to the $C_9+$ component(s). The optional toluene charge is preferably dried to minimize water entering the reaction mixture. Known methods for drying toluene are numerous, including percolation through silica gel, activated alumina, molecular sieves or other suitable substances or the use of liquid charge dryers.

When toluene and/or benzene is additionally present in the feedstock, the $C_9+$ aromatics will ordinarily constitute at least about 3 wt.% of the total feed (the balance being toluene and/or benzene) and advantageously can comprise up to 70 wt.% of the mixed feedstock. The toluene, when present in the feed, is disproportionated to aromatic compounds of high value, e.g., xylene(s) and benzene, with the more valuable xylene(s) being the predominant product(s).

In general, the process of the invention can be conducted over a wide range of conversion conditions, including a temperature of 90 to 675°C, a pressure of 100 to 14000 kPa, a hydrogen to hydrocarbon mole ratio of 0 to 10, and a weight hourly space velocity (WHSV) of 0.1 to 500.

More specifically, when the feedstock comprises a $C_9+$ alkylbenzene, especially in combination with toluene, the process is preferably conducted at a temperature of 250 to 600°C, more preferably 300 to 500°C (650 to 1000°F) and a pressure of 100 to 7000 kPa (atmospheric to 1000 psig), more preferably 445 to 7000 kPa (50 to 1000 psig). The hydrogen to hydrocarbon mole ratio is preferably 0 to 10, more preferably 0 to 3, and most preferably 0 to 2.

Where the feedstock comprises durene produced by the zeolite catalyst conversion of $C_1$-$C_4$ oxygenates, e.g., methanol and/or dimethyl ether, to gasoline, the process is preferably conducted at a temperature of 90 to 540°C (200 to 1000°F), more preferably 315 to 480°C (600 to 900°F), a pressure of 100 to 7000 kPa (0 to 1000 psig), more preferably 210 to 2170 kPa (15 to 300 psig), a mole ratio of hydrogen to hydrocarbons of 0 (i.e., no added hydrogen is present) to 10, more preferably from 1 to 3, and a weight hourly space velocity (WHSV) of 0.1 to 100, more preferably 0.1 to 10.

Useful zeolites for the present process include a zeolite having in its calcined form, an X-ray diffraction pattern with the lines listed in Table 1 below:

TABLE I

| Interplanar d-Spacing (A) | Relative Intensity, $I/I_o$ x 100 |
|---|---|
| 12.36 ± 0.4 | M-VS |
| 11.03 ± 0.2 | M-S |
| 8.83 ± 0.14 | M-VS |
| 6.18 ± 0.12 | M-VS |
| 6.00 ± 0.10 | W-M |
| 4.06 ± 0.07 | W-S |
| 3.91 ± 0.07 | M-VS |
| 3.42 ± 0.06 | VS |

more specifically the lines listed in Table II below:

TABLE II

| Interplanar d-Spacing (A) | Relative Intensity, $I/I_o$ x 100 |
|---|---|
| 30.0 ± 2.2 | W-M |
| 22.1 ± 1.3 | W |
| 12.36 ± 0.4 | M-VS |
| 11.03 ± 0.2 | M-S |
| 8.83 ± 0.14 | M-VS |
| 6.18 ± 0.12 | M-VS |

TABLE II   (continued)

| Interplanar d-Spacing (A) | Relative Intensity, I/Io x 100 |
|---|---|
| 6.00 ± 0.10 | W-M |
| 4.06 ± 0.07 | W-S |
| 3.91 ± 0.07 | M-VS |
| 3.42 ± 0.06 | VS |

and yet more specifically the lines listed in Table III below:

TABLE III

| Interplanar d-Spacing (A) | Relative Intensity, I/Io x 100 |
|---|---|
| 12.36 ± 0.4 | M-VS |
| 11.03 ± 0.2 | M-S |
| 8.83 ± 0.14 | M-VS |
| 6.86 ± 0.14 | W-M |
| 6.18 ± 0.12 | M-VS |
| 6.00 ± 0.10 | W-M |
| 5.54 ± 0.10 | W-M |
| 4.92 ± 0.09 | W |
| 4.64 ± 0.08 | W |
| 4.41 ± 0.08 | W-M |
| 4.25 ± 0.08 | W |
| 4.10 ± 0.07 | W-S |
| 4.06 ± 0.07 | W-S |
| 3.91 ± 0.07 | M-VS |
| 3.75 ± 0.06 | W-M |
| 3.56 ± 0.06 | W-M |
| 3.42 ± 0.06 | VS |
| 3.30 ± 0.05 | W-M |
| 3.20 ± 0.05 | W-M |
| 3.14 ± 0.05 | W-M |
| 3.07 ± 0.05 | W |
| 2.99 ± 0.05 | W |
| 2.82 ± 0.05 | W |
| 2.78 ± 0.05 | W |
| 2.68 ± 0.05 | W |
| 2.59 ± 0.05 | W |

Most specifically, the calcined zeolite has an X-ray diffraction pattern which includes the lines listed in Table IV below:

TABLE IV

| Interplanar d-Spacing (A) | Relative Intensity, I/Io x 100 |
|---|---|
| 30.0 ± 2.2 | W-M |
| 22.1 ± 1.3 | W |
| 12.36 ± 0.4 | M-VS |
| 11.03 ± 0.2 | M-S |
| 8.83 ± 0.14 | M-VS |
| 6.86 ± 0.14 | W-M |
| 6.18 ± 0.12 | M-VS |
| 6.00 ± 0.10 | W-M |
| 5.54 ± 0.10 | W-M |

TABLE IV   (continued)

| Interplanar d-Spacing (A) | Relative Intensity, I/Io x 100 |
|---|---|
| $4.92 \pm 0.09$ | W |
| $4.64 \pm 0.08$ | W |
| $4.41 \pm 0.08$ | W-M |
| $4.25 \pm 0.08$ | W |
| $4.10 \pm 0.07$ | W-S |
| $4.06 \pm 0.07$ | W-S |
| $3.91 \pm 0.07$ | M-VS |
| $3.75 \pm 0.06$ | W-M |
| $3.56 \pm 0.06$ | W-M |
| $3.42 \pm 0.06$ | VS |
| $3.30 \pm 0.05$ | W-M |
| $3.20 \pm 0.05$ | W-M |
| $3.14 \pm 0.05$ | W-M |
| $3.07 \pm 0.05$ | W |
| $2.99 \pm 0.05$ | W |
| $2.82 \pm 0.05$ | W |
| $2.78 \pm 0.05$ | W |
| $2.68 \pm 0.05$ | W |
| $2.59 \pm 0.05$ | W |

These values were determined by standard techniques. The radiation was the K-alpha doublet of copper and a diffractometer equipped with a scintillation counter and an associated computer was used. The peak heights, I, and the positions as a function of 2 theta, where theta is the Bragg angle, were determined using algorithms on the computer associated with the diffractometer. From these, the relative intensities, 100 $I/I_o$, where $I_o$ is the intensity of the strongest line or peak, and d (obs.) the interplanar spacing in Angstroms Units (A), corresponding to the recorded lines, were determined. In Tables I-IV, the relative intensities are given in terms of the symbols W-weak, M=medium, S=strong and VS=very strong. In terms of intensities, these may be generally designated as follows:

$$W = 0 - 20$$

$$M = 20 - 40$$

$$S = 40 - 60$$

$$VS = 60 - 100$$

It should be understood that these X-ray diffraction patterns are characteristic of all species of the zeolite. The sodium form as well as other cationic forms reveal substantially the same pattern with some minor shifts in interplanar spacing and variation in relative intensity. Other minor variations can occur depending on the Y to X, e.g., silicon to aluminum, mole ratio of the particular sample, as well as its degree of thermal treatment.

The zeolite of Tables I - IV generally has a composition involving the molar relationship:

$$X_2O_3:(n)YO_2,$$

wherein X is a trivalent element, such as aluminum, boron, iron and/or gallium, preferably aluminum, Y is a tetravalent element such as silicon and/or germanium, preferably silicon, and n is at least 10, usually from 10 to 150, more usually from 10 to 60, and even more usually from 20 to 40. In the as-synthesized form, the zeolite has a formula, on an anhydrous basis and in terms of moles of oxides per n moles of $YO_2$, as follows:

$$(0.005\text{-}0.1)Na_2O{:}(1\text{-}4)R{:}X_2O_3{:}nYO_2$$

wherein R is an organic component. The Na and R components are associated with the zeolite as a result of their presence during crystallization, and are easily removed by post-crystallization methods hereinafter more particularly described.

The above zeolite is thermally stable and exhibits high surface area (greater than 400 $m^2$/gm as measured by the BET [Bruenauer, Emmet and Teller] test). In addition, the zeolite normally exhibits equilibrium adsorption capacities greater than 4.5 wt.% for cyclohexane vapor, greater than 10 wt.% for n-hexane vapor and preferably greater than 10 wt.% for water vapor. As is evident from the above formula, the zeolite is synthesized nearly free of Na cations. It can therefore be used as a catalyst with acid activity without an exchange step. To the extent desired, however, the original sodium cations of the as-synthesized zeolite and the other zeolites useful in the present process can be replaced in accordance with techniques well known in the art, at least in part, by ion exchange with other cations. Preferred replacing cations include metal ions, hydrogen ions, hydrogen precursor, e.g., ammonium, ions and mixtures thereof. Particularly preferred cations are those which tailor the catalytic activity for transalkylation/disproportionation. These include hydrogen, rare earth metals and metals of Groups IIA, IIIA, IVA, IB, IIB, IIIB, IVB and VIII of the Periodic Table of the Elements.

The zeolite defined in Tables I - IV can be prepared from a reaction mixture containing sources of alkali or alkaline earth metal (M), e.g., sodium or potassium, cation, an oxide of trivalent element X, e.g, aluminum, an oxide of tetravalent element Y, e.g., silicon, an organic (R) directing agent in the form of hexamethyleneimine and water, said reaction mixture having a composition, in terms of mole ratios of oxides, within the following ranges:

| Reactants | Useful | Preferred |
|---|---|---|
| $YO_2/X_2O_3$ | 10 - 60 | 10-40 |
| $H_2O/YO_2$ | 5-100 | 10-50 |
| $OH^-/YO_2$ | 0.01-1.0 | 0.1-0.5 |
| $M/YO_2$ | 0.01-2.0 | 0.1-1.0 |
| $R/YO_2$ | 0.05-1.0 | 0.1-0.5 |

In a preferred synthesis method the $YO_2$ reactant contains a substantial amount of solid $YO_2$, e.g., at least about 30 wt.% solid $YO_2$. Where $YO_2$ is silica, the use of a silica source containing at least about 30 wt.% solid silica, e.g., Ultrasil (a precipitated, spray dried silica containing about 90 wt.% silica) or HiSil (a precipitated hydrated $SiO_2$ containing about 87 wt.% silica, about 6 wt.% free $H_2O$ and about 4.5 wt.% bound $H_2O$ of hydration and having a particle size of about 0.02 micron) favors crystal formation from the above mixture. If another source of oxide of silicon, e.g., Q-Brand (a sodium silicate comprised of about 28.8 wt.% of $SiO_2$, 8.9 wt.% $Na_2O$ and 62.3 wt.% $H_2O$) is used, crystallization may yield little if any of the desired crystalline material and impurity phases of other crystal structures may be produced. Preferably, therefore, the $YO_2$, e.g., silica, source contains at least about 30 wt.% solid $YO_2$, e.g., silica, and more preferably at least about 40 wt.% solid $YO_2$, e.g., silica.

Crystallization can be carried out at either static or stirred conditions in a suitable reactor vessel such as, e.g., polypropylene jars or teflon-lined or stainless steel autoclaves. Suitable crystallization conditions include a temperature of 80°C to 225°C for a time of 25 hours to 60 days. Thereafter, the crystals are separated from the liquid and recovered.

Synthesis is facilitated by the presence of at least about 0.01 percent, preferably about 0.10 percent and still more preferably about 1 percent, seed crystals (based on total weight) of the required crystalline product.

The zeolite conversion catalysts used in the process of the invention are conveniently employed in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such component can be introduced in the catalyst composition by way of co-crystallization, exchanged into the composition to the extent a Group IIIA element, e.g., aluminum, is in the structure, impregnated therein or intimately physically admixed therewith. Such component can be impregnated in, or on, the zeolite such as, for example, by, in the case of platinum, treating the zeolite with a solution containing a platinum metal-containing ion. Thus, suitable platinum compounds for this purpose include chloroplatinic acid, platinous chloride and various compounds containing the platinum amine complex.

Prior the use in the process of the invention, the selected zeolite catalyst is preferably combined with another material which is resistant to the temperatures and other conditions employed in the process. Such materials include active and inactive materials and synthetic or naturally occurring zeolites as well as inorganic materials such as clays, silica and/or metal oxides such as alumina. The latter may be either naturally occurring or in the form of gelatinous precipitates or gels including mixtures of silica and metal oxides. Use of a material in conjunction with the catalyst zeolite, i.e., combined therewith or present during its synthesis, which itself is catalytically active may change the conversion and/or selectivity of the catalyst. Inactive materials suitably serve as diluents to control the amount of conversion so that transalkylated/ disproportionated products can be obtained economically and orderly without employing other means for controlling the rate of reaction. These materials may be incorporated into naturally occurring clays, e.g., bentonite and kaolin, to improve the crush strength of the catalyst under commercial alkylation operating conditions, Said materials, i.e., clays, oxides, etc., function as binders for the catalyst. It is desirable to provide a catalyst having good crush strength because in commercial use, it is desirable to prevent the catalyst from breaking down into powder-like materials. These clay binders have been employed normally only for the purpose of improving the crush strength of the catalyst.

Naturally occurring clays which can be composited with the zeolite catalyst herein include the montmorillonite and kaolin family, which families include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification. Binders useful for compositing with zeolite also include inorganic oxides, notably alumina.

In addition to the foregoing materials, the zeolite catalyst can be composited with a porous matrix material such as silica-alumina, silica-magnesia, silica-zirconia, silica-thoria, silica-beryllia, silica-titania as well as ternary compositions such as silica-alumina-thoria, silica-alumina-zirconia, silica-alumina-magnesia and silica-magnesia-zirconia. It may also be advantageous to provide at least a part of the foregoing matrix materials in colloidal form so as to facilitate extrusion of the bound catalyst component(s).

The relative proportions of finely divided crystalline material and inorganic oxide matrix vary widely, with the crystal content ranging from 1 to 90 percent by weight and more usually, particularly when the composite is prepared in the form of beads, in the range of 2 to 80 weight percent of the composite.

The stability of the zeolite catalyst may be increased by steaming, with suitable steam stabilization conditions include contacting the catalyst with, e.g., 5-100% steam at a temperature of at least 300°C (e.g., 300-650°C) for at least one hour (e.g., 1-200 hours) at a pressure of 100-2,500 kPa. In a more particular embodiment, the catalyst can be made to undergo steaming with 75-100% steam at 315-500°C and atmospheric pressure for 2-25 hours.

The invention will now be more fully described with reference to the following Examples and the accompanying drawings in which:

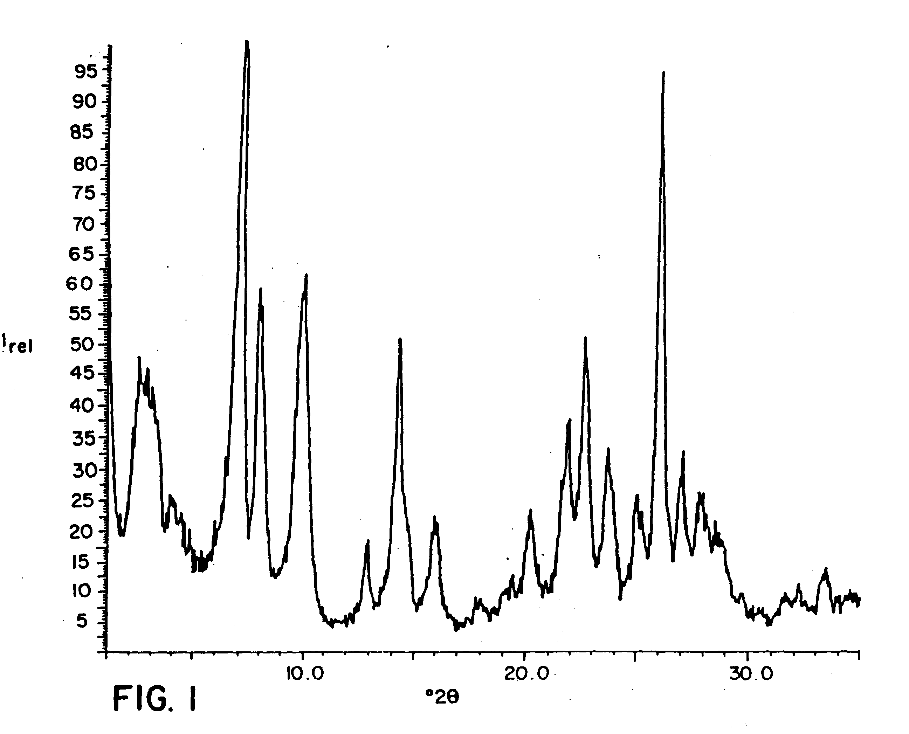

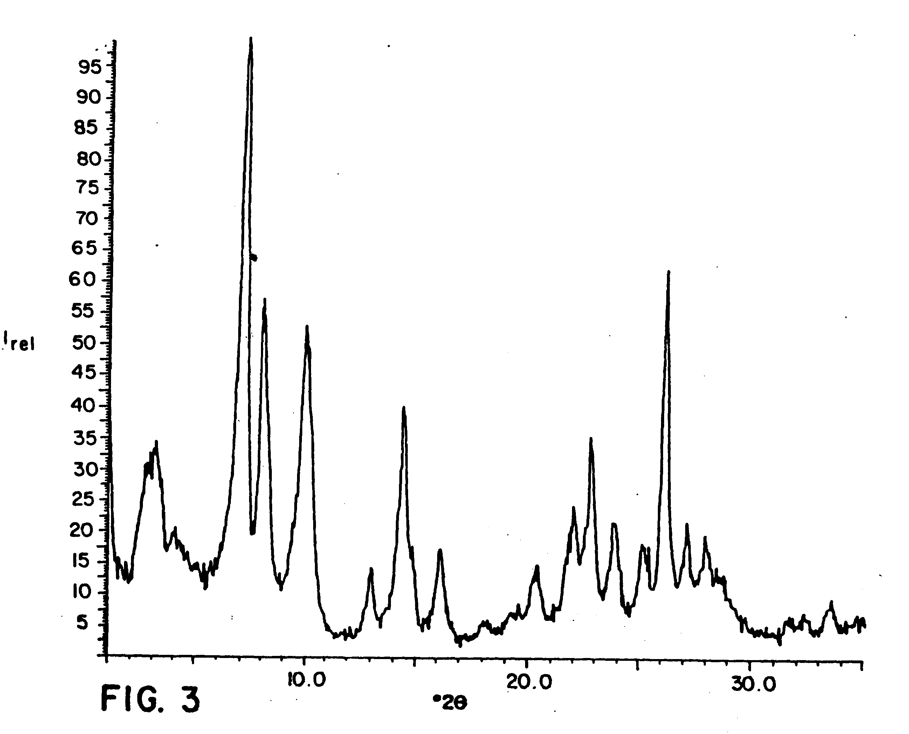

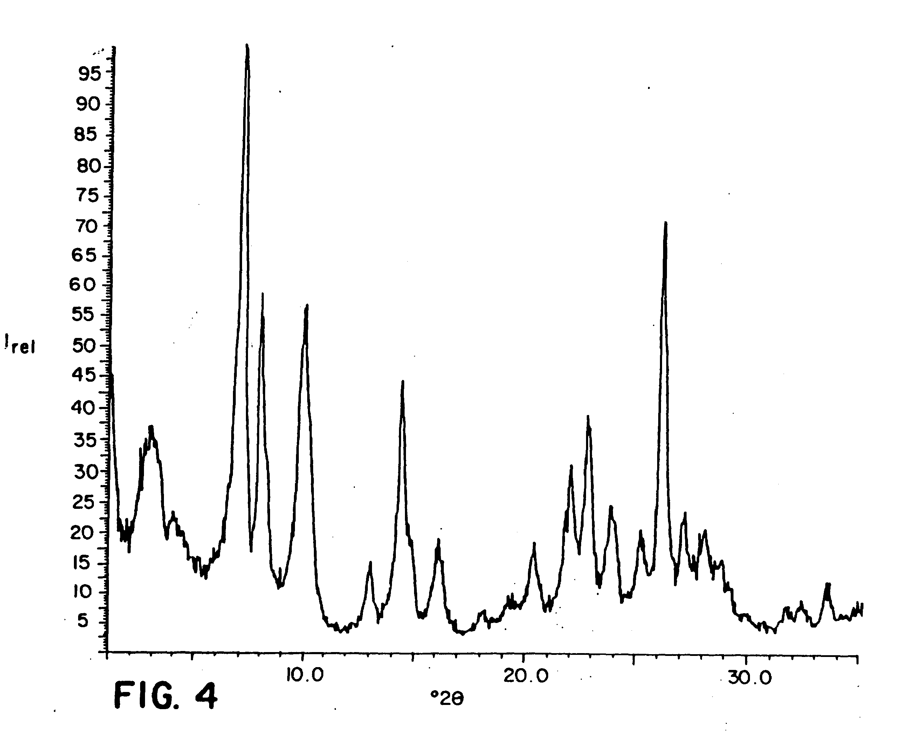

Figures 1-5 are X-ray diffraction patterns of the calcined crystalline material products of Examples 1, 3, 4, 5 and 7, respectively.

In the Examples, whenever sorption data are set forth for comparison of sorptive capacities for water, cyclohexane and/or n-hexane, they were Equilibrium Adsorption values determined as follows:

A weighed sample of the calcined adsorbent was contacted with the desired pure adsorbate vapor in an adsorption chamber, evacuated to less than 1 mm Hg and contacted with 1.6 kPa (12 Torr) of water vapor or 5.3 kPa (40 Torr) of n-hexane or 5.3 kPa (40 Torr) of cyclohexane vapor, pressures less than the vapor-liquid equilibrium pressure of the respective adsorbate at 90°C. The pressure was kept constant (within about ± 0.5 mm Hg) by addition of adsorbate vapor controlled by a manostat during the adsorption period, which did not exceed 8 hours. As adsorbate was adsorbed by the zeolite, the decrease in pressure caused the manostat to open a valve which admitted more adsorbate vapor to the chamber to restore the above control pressures. Sorption was complete when the pressure change was not sufficient to activate the manostat. The increase in weight was calculated as the adsorption capacity of the sample in g/100 g of calcined adsorbent.

When Alpha Value is examined, it is noted that the Alpha Value is an approximate indication of the catalytic cracking activity of the catalyst compared to a standard catalyst and it gives the relative rate constant (rate of normal hexane conversion per volume of catalyst per unit time). It is based on the activity of a highly active silica-alumina cracking catalyst taken as an Alpha of 1 (Rate Constant = 0.016 sec$^{-1}$). The Alpha Test which was used herein is described in J. Catalysis, 61, pp. 390-396 (1980). It is noted that intrinsic rate constants for many acid-catalyzed reactions are proportional to the Alpha Value for a particular zeolite catalyst, i.e., the rates for toluene disproportionation, xylene isomerization, alkene conversion and methanol conversion (see "The Active Side of Acidic Aluminosilicate Catalysts, " Nature, Vol. 309, No. 5969, pp. 589-591, 14 June 1984).

EXAMPLE 1

1 part of sodium aluminate (43.5% $Al_2O_3$, 32.2% $Na_2O$, 25.6% $H_2O$) was dissolved in a solution containing 1 part of 50% NaOH solution and 103.13 parts $H_2O$. To this was added 4.50 parts hexamethyleneimine. The resulting solution was added to 8.55 parts of Ultrasil, a precipitated, spray-dried silica (about 90% $SiO_2$).

The reaction mixture had the following composition, in mole ratios:

$$SiO_2/Al_2O_3 = 30.0$$

$$OH^-/SiO_2 = 0.18$$

$$H_2O/SiO_2 = 44.9$$

$$Na/SiO_2 = 0.18$$

$$R/SiO_2 = 0.35$$

where R is hexamethyleneimine.

The mixture was crystallized in a stainless steel reactor, with stirring, at 150°C for 7 days. The crystalline product was filtered, washed with water and dried at 120°C. After a 20 hour calcination at 538°C, the X-ray diffraction pattern contained the major lines listed in Table V. Figure 1 shows the X-ray diffraction pattern of the calcined product. The sorption capacities of the calcined material were measured to be:

| $H_2O$ | 15.2 wt.% |
| Cyclohexane | 14.6 wt.% |
| n-Hexane | 16.7 wt.% |

The surface area of the calcined crystalline material was measured to be 494 $m^2/g$.

The chemical composition of the uncalcined material was determined to be as follows:

| Component | wt.% |
|---|---|
| $SiO_2$ | 66.9 |
| $Al_2O_3$ | 5.40 |
| Na | 0.03 |
| N | 2.27 |
| Ash | 76.3 |
| $SiO_2/Al_2O_3$, mole ratio | -21.1 |

TABLE V

| Degrees 2-Theta | Interplanar d-Spacing (A) | $I/I_o$ |
|---|---|---|
| 2.80 | 31.55 | 25 |
| 4.02 | 21.98 | 10 |
| 7.10 | 12.45 | 96 |
| 7.95 | 11.12 | 47 |
| 10.00 | 8.85 | 51 |
| 12.90 | 6.86 | 11 |
| 14.34 | 6.18 | 42 |
| 14.72 | 6.02 | 15 |
| 15.90 | 5.57 | 20 |
| 17.81 | 4.98 | 5 |
| 20.20 | 4.40 | 20 |
| 20.91 | 4.25 | 5 |
| 21.59 | 4.12 | 20 |
| 21.92 | 4.06 | 13 |

TABLE V  (continued)

| Degrees 2-Theta | Interplanar d-Spacing (A) | $I/I_o$ |
|---|---|---|
| 22.67 | 3.92 | 30 |
| 23.70 | 3.75 | 13 |
| 24.97 | 3.57 | 15 |
| 25.01 | 3.56 | 20 |
| 26.00 | 3.43 | 100 |
| 26.69 | 3.31 | 14 |
| 27.75 | 3.21 | 15 |
| 28.52 | 3.13 | 10 |
| 29.01 | 3.08 | 5 |
| 29.71 | 3.01 | 5 |
| 31.61 | 2.830 | 5 |
| 32.21 | 2.779 | 5 |
| 33.35 | 2.687 | 5 |
| 34.61 | 2.592 | 5 |

EXAMPLE 2

A portion of the calcined crystalline product of Example 1 was tested in the Alpha Test and was found to have an Alpha Value of 224.

EXAMPLES 3-5

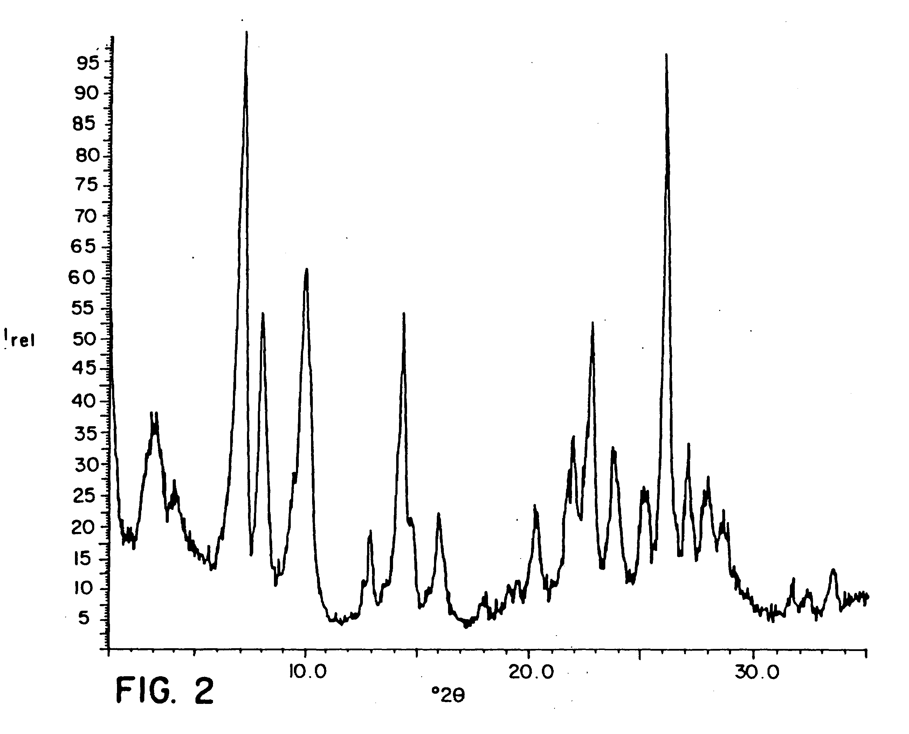

Three separate synthesis reaction mixtures were prepared with compositions indicated in Table VI. The mixtures were prepared with sodium aluminate, sodium hydroxide, Ultrasil, hexamethyleneimine (R) and water. The mixtures were maintained at 150°C, 143°C and 150°C, respectively, for 7, 8 and 6 days respectively in stainless steel autoclaves at autogenous pressure. Solids were separated from any unreacted components by filtration and then water washed, followed by drying at 120°C. The product crystals were analyzed by X-ray diffraction, sorption, surface area and chemical analyses. The results of the sorption, surface area and chemical analyses are presented in Table VI and the X-ray diffraction patterns are presented in Figures 2, 3 and 4, respectively. The sorption and surface area measurements were of the calcined product.

TABLE VI

| Example | 3 | 4 | 5 |
|---|---|---|---|
| Synthesis Mixture, mole ratios | | | |
| $SiO_2/Al_2O_3$ | 30.0 | 30.0 | 30.0 |
| $OH^-/SiO_2$ | 0.18 | 0.18 | 0.18 |
| $H_2O/SiO_2$ | 19.4 | 19.4 | 44.9 |
| $Na/SiO_2$ | 0.18 | 0.18 | 0.18 |
| $R/SiO_2$ | 0.35 | 0.35 | 0.35 |
| Product Composition, Wt.% | | | |
| $SiO_2$ | 64.3 | 68.5 | 74.5 |
| $Al_2O_3$ | 4.85 | 5.58 | 4.87 |
| Na | 0.08 | 0.05 | 0.01 |
| N | 2.40 | 2.33 | 2.12 |
| Ash | 77.1 | 77.3 | 78.2 |
| $SiO_2/Al_2O_3$, mole ratio | 22.5 | 20.9 | 26.0 |
| Adsorption, Wt.% | | | |
| $H_2O$ | 14.9 | 13.6 | 14.6 |
| Cyclohexane | 12.5 | 12.2 | 13.6 |

TABLE VI   (continued)

| Example | 3 | 4 | 5 |
|---|---|---|---|
| Adsorption, Wt.% | | | |
| n-Hexane | 14.6 | 16.2 | 19.0 |
| Surface Area, $m^2/g$ | 481 | 492 | 487 |

## EXAMPLE 6

Quantities of the calcined (538°C for 3 hours) crystalline silicate products of Examples 3, 4 and 5 were tested in the Alpha Test and found to have Alpna Values of 227, 180 and 187, respectively.

## EXAMPLE 7

To demonstrate a further preparation of the present zeolite, 4.49 parts of hexamethyleneimine was added to a solution containing 1 part of sodium aluminate, 1 part of 50% NaOH solution and 44.19 parts of $H_2O$. To the combined solution were added 8.54 parts of Ultrasil silica. The mixture was crystallized with agitation at 145°C for 59 hours and the resultant product was water washed and dried at 120°C.

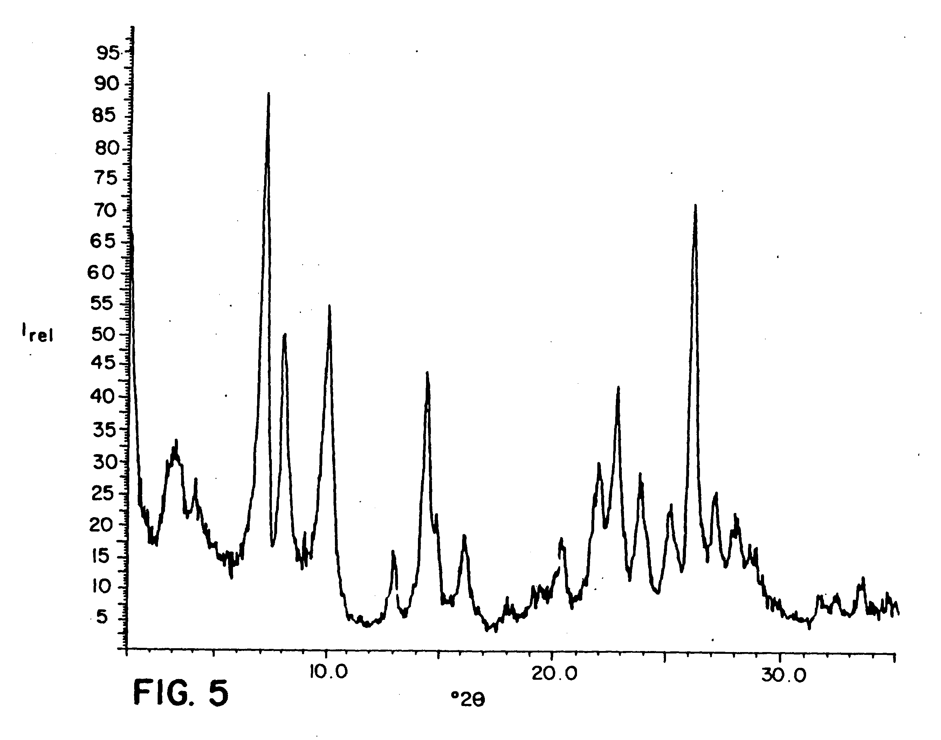

The X-ray diffraction pattern of the dried product crystals is presented in Figure 5. Product chemical composition, surface area and adsorption analyses results were as set forth in Table VII:

TABLE VII

| Product Composition (uncalcined) | |
|---|---|
| C | 12.1 wt.% |
| N | 1.98 wt.% |
| Na | 640 ppm |
| $Al_2O_3$ | 5.0 wt.% |
| $SiO_2$ | 74.9 wt.% |
| $SiO_2/Al_2O_3$, mole ratio | 25.4 |
| Adsorption, wt.% | |
| Cyclohexane | 9.1 |
| N-Hexane | 14.9 |
| $H_2O$ | 16.8 |
| Surface Area, $m^2/g$ | 479 |

## EXAMPLE 8

25g grams of solid crystal product from Example 7 were calcined in a flowing nitrogen atmospheres at 538°C for 5 hours, followed by purging with 5% oxygen gas (balance $N_2$) for another 16 hours at 538°C.

Individual 3g samples of the calcined material were ion-exchanged with 100 ml of 0.1N TEABr, TPABr and $LaCl_3$ solution separately. Each exchange was carried out at ambient temperature for 24 hours and repeated three times. The exchanged samples were collected by filtration, water-washed to be halide-free and dried. The compositions of the exchanged samples are tabulated below.

| Exchange Ions Ionic Composition, wt.% | TEA | TPA | La |
|---|---|---|---|
| Na | 0.095 | 0.089 | 0.063 |
| N | 0.30 | 0.38 | 0.03 |
| C | 2.89 | 3.63 | - |
| La | - | - | 1.04 |

### EXAMPLE 9

The La-exchanged sample from Example 8 was sized to 14 to 25 mesh and then calcined in air at 538°C for 3 hours. The calcined material had an Alpha Value of 173.

### EXAMPLE 10

The calcined sample La-exchanged material from Example 9 was severely steamed at 649°C in 100% steam for 2 hours. The steamed sample had an Alpha Value of 22, demonstrating that the zeolite had very good stability under severe hydrothermal treatment.

### EXAMPLE 11

This example illustrates the preparation of the present zeolite where X in the general formula, <u>supra</u>, is boron. Boric acid, 2.59 parts, was added to a solution containing 1 part of 45% KOH solution and 42.96 parts $H_2O$. To this was added 8.56 parts of Ultrasil silica, and the mixture was thoroughly homogenized. A 3.88 parts quantity of hexamethyleneimine was added to the mixture.

The reaction mixture had the following composition in mole ratios:

$$SiO_2/B_2O_3 = 6.1$$

$$OH^-/SiO_2 = 0.06$$

$$H_2O/SiO_2 = 19.0$$

$$K/SiO_2 = 0.06$$

$$R/SiO_2 = 0.30$$

where R is hexamethyleneimine.

The mixture was crystallized in a stainless steel reactor, with agitation, at 150°C for 8 days. The crystalline product was filtered, washed with water and dried at 120°C. A portion of the product was calcined for 6 hours at 540°C and found to have the following sorption capacities:

| | |
|---|---|
| $H_2O$ | 11.7 wt.% |
| Cyclohexane | 7.5 wt.% |
| n-Hexane | 11.4 wt.% |

The surface area of the calcined crystalline material was measured (BET) to be 405m$^2$/g.

The chemical composition of the uncalcined material was determined to be as follows:

| | |
|---|---|
| N | 1.94 wt.% |
| Na | 175 ppm |
| K | 0.60 wt.% |
| Boron | 1.04 wt.% |
| $Al_2O_3$ | 920 ppm |
| $SiO_2$ | 75.9 wt.% |
| Ash | 74.11 wt.% |
| $SiO_2/Al_2O_3$, molar ratio = | 1406 |
| $SiO_2/(Al+B)_2O_3$, molar ratio = | 25.8 |

## EXAMPLE 12

A portion of the calcined crystalline product of Example 11 was treated with $NH_4Cl$ and again calcined. The final crystalline product was tested in the Alpha Test and found to have an Alpha Value of 1.

## EXAMPLE 13

This example illustrates another preparation of the zeolite in which X of the general formula, <u>supra</u>, is boron. Boric acid, 2.23 parts, was added to a solution of 1 part of 50% Sam solution and 73.89 parts $H_2O$. To this solution was added 15.29 parts of HiSil silica followed by 6.69 parts of hexamethyleneimine. The reaction mixture had the following composition in mole ratios:

$$SiO_2/B_2O_3 = 12.3$$

$$OH^-/SiO_2 = 0.056$$

$$H_2O/SiO_2 = 18.6$$

$$K/SiO_2 = 0.056$$

$$R/SiO_2 = 0.30$$

where R is hexamethyleneimine.

The mixture was crystallized in a stainless steel reactor, with agitation, at 300°C for 9 days. The crystalline product was filtered, washed with water and dried at 120°C. The sorption capacities of the calcined material (6 hours at 540°C) were measured:

| | |
|---|---|
| $H_2O$ | 14.4 wt.% |
| Cyclohexane | 4.6 wt.% |
| n-Hexane | 14.0 wt.% |

The surface area of the calcined crystalline material was measured to be $438 m^2/g$.

The chemical composition of the uncalcined material was determined to be as follows:

| Component | Wt.% |
|---|---|
| N | 2.48 |
| Na | 0.06 |
| Boron | 0.83 |
| $Al_2O_3$ | 0.50 |
| $SiO_2$ | 73.4 |
| $SiO_2/Al_2O_3$, molar ratio = | 249 |
| $SiO_2/(Al+B)_2O_3$, molar ratio = | 28.2 |

## EXAMPLE 14

A portion of the calcined crystalline product of Example 13 was tested in the Alpha Test and found to have an Alpha Value of 5.

## EXAMPLES 15 and 16

These examples illustrate the catalytic conversion of a mixture of toluene and a $C_9+$ aromatics feed with a zeolite of Tables I - IV (Example 15) and compare the performance of this zeolite with that of ZSM-5, i.e., a catalyst which is outside the scope of this invention, (Example 16).

The zeolite of the invention was prepared by adding 4.49 parts hexamethyleneimine to a mixture containing 1.00 part sodium aluminate, 1.00 part 50% NaOH, 8.54 parts Ultrasil VN3 and 44.19 parts deionized $H_2O$. The reaction mixture was heated to 143°C (290°F) and stirred in an autoclave at that temperature for crystallization. After full crystallinity was achieved, the majority of the hexamethyleneimine was removed from the autoclave by controlled distillation and the zeolite crystals separated from the remaining liquid by filtration, washed with deionized $H_2O$ and dried. A 65 wt.% zeolite/35 wt.% $Al_2O_3$ catalyst composition was prepared from the zeolite by extrusion. The material was then dried overnight at 120°C (250°F), calcined at 480°C (900°F) for three hours in 3v/v/min $N_2$, then treated with 50 vol. % air/50 vol.% $N_2$ at 3v/v/min, also at 480°C (900°F) for one hour. The calcination was completed by raising the temperature to 540°C (1000°F) at 3°C (5°F)/min and finally switching to 100% air (3v/v/min) and holding at this temperature for three hours. A similar process was used to prepare the ZSM-5 catalyst.

The properties of the zeolite catalyst compositions are set forth in Table VIII as follows:

TABLE VIII

|  | Zeolite of Tables I-IV | ZSM-5 |
|---|---|---|
| $SiO_2/Al_2O_3$, molar | 25 | 26 |
| Alpha Value | 215 | 423 |
| Sodium, ppm | 630 | 135 |
| Surface area, $m^2/g$ | 451 | 325 |
| Real density, g/cc | 2.57 | 2.64 |
| Particle density, g/cc | 0.82 | 0.87 |
| Pore volume, cc/g | 0.83 | 0.77 |

The $C_9+$ aromatics feed had the composition set forth in Table IX as follows:

TABLE IX

|  | Wt.% | Mole% |
|---|---|---|
| Ethylbenzene | 0.02 | 0.02 |
| p-Xylene | 0.14 | 0.16 |
| o-Xylene | 0.36 | 0.41 |
| $C_9$ Aromatics | 83.29 | 84.60 |
| TMB (trimethylbenzene) | 39.05 | 39.66 |
| MEB (methylethylbenzene) | 37.38 | 37.97 |
| $C_{10}+$ Aromatics | 15.53 | 14.28 |
| DEB (dimethylethylbenzene) | 6.59 | 6.00 |
| DMEB | 4.51 | 4.10 |
|  |  |  |
| Total Xylenes | 0.50 | 0.58 |
| Total Aromatics | 99.34 | 99.49 |
| Total Non-Aromatics | 0.00 | 0.00 |

The total feed compositions are shown in Table X as follows:

TABLE X

| Total Feed Composition | | |
|---|---|---|
|  | Example 15 | Example 16 |
| Toluene | 67.45 | 66.86 |
| $C_9+$ | 31.84 | 31.88 |
| $C_9-$ | 0.71 | 1.26 |

Each of the experiments was conducted in a stainless steel reactor having an external diameter of 1 cm at 4240 kPa (600 psig), 4 hr$^{-1}$ weight hourly space velocity (based on zeolite) and a hydrogen/hydrocarbon mole ratio of 2. The toluene was initially passed over the catalyst in each instance at a temperature required to maintain 48 ± 1 wt.% toluene conversion.

Table XI below sets forth the reaction conditions and the product distributions:

TABLE XI

| Product Results | | |
|---|---|---|
| | Example 15 | Example 16 |
| **Conditions** | | |
| Temperature [1], °F(°C) | 885 (474) | 750 (399) |
| Toluene Conversion, wt.% | 48[2] | 48[2] |
| C$_9$+ Conversion, wt.% | 66 | 62 |
| **Product Distributions** | | |
| C$_5$-, wt.% | 7.53 | 7.16 |
| Benzene | 16.94 | 19.86 |
| Toluene | 39.63 | 38.31 |
| Ethylbenzene | 1.37 | 1.34 |
| p-Xylene | 5.74 | 5.04 |
| m-Xylene | 12.26 | 10.93 |
| o-Xylene | 5.59 | 4.86 |
| Xylene/Benzene mole ratio | 1.02 | 0.77 |
| C$_9$+, wt.% | 10.94 | 12.50 |

[1] Initial temperature required to maintain 48 ± 1 wt% toluene conversion.

[2] Initial toluene conversion which became 41 wt% and 43 wt%, respectively in Examples 15 and 16 after C$_9$+ feed addition.

It is observed from these experiments that the present process, exemplified by Example 15, provides increased product xylene when compared to the experiment conducted with a similar feed but using ZSM-5 catalyst.

An additional benefit provided by the present process is the increased C$_9$+ conversion (Example 17: 66 wt.% compared to Example 18: 62 wt.%).

EXAMPLES 17 and 18

The process of Examples 15 and 16 was repeated with feed compositions shown in Table XII below and with the weight hourly space velocity increased to 6 hr$^{-1}$. Product distributions from these experiments are presented in Table XIII below.

TABLE XII

| | Example 17 | Example 18 |
|---|---|---|
| Toluene | 67.45 | 66.55 |
| C$_9$+ | 31.84 | 32.17 |
| C$_9$- | 0.71 | 1.28 |

TABLE XIII

| | Example 17 | Example 18 |
|---|---|---|
| Catalyst | Zeolite of Tables I-IV | ZSM-5 |
| **Product Distribution** | | |
| C$_5$-, wt% | 5.33 | 6.09 |
| Benzene, wt% | 15.71 | 18.51 |
| Toluene, wt% | 41.90 | 39.61 |

TABLE XIII   (continued)

| | Example 17 | Example 18 |
|---|---|---|
| Catalyst | Zeolite of Tables I-IV | ZSM-5 |
| Product Distribution | | |
| Ethylbenzene, wt% | 1.66 | 1.40 |
| p-Xylene, wt% | 5.45 | 4.97 |
| m-Xylene, wt% | 11.55 | 10.61 |
| o-Xylene, wt% | 5.31 | 4.62 |
| Xylene/Benzene, mole ratio | 1.04 | 0.80 |
| $C_9+$, wt% | 13.09 | 14.19 |

The results of Examples 17 and 18 again demonstrate the unexpected improvement of the present invention. The product mole ratio of xylene/benzene was 1.04 for Example 17 compared to only 0.80 for Example 18.

EXAMPLES 19 - 33

These examples illustrate the use of the zeolite of the invention in the conversion of durene. The zeolite was prepared by adding a 4.49 parts quantity of hexamethyleneimine to a mixture containing 1.00 part sodium aluminate, 1.00 part 50% NaOH, 8.54 parts Ultrasil VN3 and 44.19 parts deionized $H_2O$. The reaction mixture was heated to 143°C (290°F) and stirred in an autoclave at that temperature for crystallization. After full crystallinity was achieved, the majority of the hexamethyleneimine was removed from the autoclave by controlled distillation and the zeolite crystals separated from the remaining liquid by filtration, washed with deionized $H_2O$ and dried. A portion of the zeolite crystals was combined with $Al_2O_3$ to form a mixture of 65 parts, by weight, zeolite and 35 parts $Al_2O_3$. Water was added to this mixture to allow the resulting catalyst to be formed into extrudates. The catalyst was activated by calcining at 480°C (900°F) in 3v/v/min nitrogen for three hours, then treated with 50 vol.% air/50 vol.% $N_2$ at 3v/v/min, also at 480°C (900°F). The calcination was completed by raising the temperature to 540°C (1000°F) at 5°F/min and finally switching to 100% air (3v/v/min) and holding at 540°C (1000°F) for three hours.

The feed employed was a mixture of approximately 25 wt. % durene and 75 wt.% benzene. The temperature, pressure, hydrogen to hydrocarbon mole ratio and WHSV (based on zeolite) conditions and the product distributions obtained in each example are set forth in Table XVII as follows:

EP 0 457 853 B1

TABLE XVII

| | Feed | 19 | 20 | 21 | 22 | 23 | 24 | 25 | 26 | 27 | 28 | 29 | 30 | 31 | 32 | 33 |
|---|---|---|---|---|---|---|---|---|---|---|---|---|---|---|---|---|
| **CONDITIONS** | | | | | | | | | | | | | | | | |
| Temperature, °F(C°) | --- | 601(316) | 700(371) | 700(371) | 700(371) | 801(427) | 802(428) | 802(428) | 802(428) | 802(428) | 802(428) | 802(428) | 802(428) | 849(454) | 849(454) | 849(454) |
| WHSV (zeolite) | --- | 4.3 | 4.0 | 4.0 | 4.1 | 4.1 | 4.0 | 4.1 | 4.1 | 4.1 | 4.1 | 4.1 | 4.1 | 4.0 | 4.0 | 4.0 |
| Pressure, psig (kpa) | | 600(4240) | 600(4240) | 600(4240) | 600(4240) | 630(4445) | 630(4445) | 630(4445) | 640(4514) | 620(4376) | 620(4376) | 630(4445) | 630(4445) | 620(4376) | 620(4376) | 620(4376) |
| $H_2$/HC mole ratio | --- | 2 | 2 | 2 | 2 | 2 | 2 | 2 | 2 | 2 | 2 | 2 | 2 | 2 | 2 | |
| **PRODUCT DISTRIBUTION, wt.%** | | | | | | | | | | | | | | | | |
| $C_5^-$ | --- | 0.2 | 0.2 | 0.2 | 0.1 | 1.2 | 0.9 | 0.9 | 0.7 | 1.1 | 0.8 | 0.9 | 0.7 | 1.6 | 1.5 | 1.3 |
| Benzene | 75.2 | 70.6 | 69.2 | 70.6 | 71.6 | 66.1 | 63.9 | 63.7 | 66.0 | 66.3 | 63.9 | 64.0 | 69.0 | 62.8 | 63.5 | 61.4 |
| Toluene | --- | 5.3 | 5.9 | 4.4 | 3.6 | 10.6 | 9.9 | 9.3 | 8.7 | 8.3 | 8.7 | 8.7 | 7.6 | 13.1 | 12.4 | 12.5 |
| Ethylbenzene | --- | 0.1 | 0.2 | 0.2 | 0.2 | 0.9 | 0.8 | 0.7 | 0.6 | 0.6 | 0.6 | 0.6 | 0.5 | 1.0 | 1.0 | 1.0 |
| Xylenes | --- | 1.5 | 1.6 | 1.1 | 0.9 | 2.0 | 3.7 | 3.5 | 3.3 | 3.1 | 3.3 | 3.3 | 2.7 | 4.8 | 4.5 | 4.6 |
| Ethyltoluenes | --- | --- | 0.1 | 0.1 | 0.1 | 0.2 | 0.1 | 0.1 | 0.1 | 0.1 | 0.1 | 0.1 | 0.1 | 0.1 | 0.1 | 0.1 |
| Trimethylbenzenes | 0.2 | 5.9 | 7.2 | 5.7 | 4.7 | 8.5 | 8.8 | 8.7 | 8.1 | 7.8 | 8.6 | 8.6 | 7.2 | 8.1 | 7.9 | 8.5 |
| Durene | 24.6 | 14.5 | 13.7 | 15.5 | 16.5 | 9.0 | 10.3 | 11.3 | 10.8 | 10.9 | 12.0 | 11.9 | 10.6 | 7.3 | 7.8 | 9.1 |
| Unknown Fractions | --- | 1.9 | 1.9 | 2.2 | 2.3 | 1.5 | 1.6 | 1.8 | 1.7 | 1.8 | 2.0 | 1.9 | 1.6 | 1.2 | 1.3 | 1.5 |
| | | 100 | 100 | 100 | 100 | 100 | 100 | 100 | 100 | 100 | 100 | 100 | 100 | 100 | 100 | 100 |
| % Durene Converted | | 41.1 | 44.3 | 37.0 | 32.9 | 63.4 | 58.1 | 54.1 | 56.1 | 55.7 | 51.2 | 51.6 | 56.9 | 70.3 | 68.3 | 63.0 |

The data show that the zeolite of Tables I -IV is effective in converting the model durene/benzene feed. Between 315 -427°C (600 - 800°F), a range of durene conversions of from 33-70% was observed. Selectivities for toluene ranged from 3-13% and selectivities for xylenes ranged from 1-5%.

**Claims**

1. A process for converting a feedstock containing at least one mononuclear aromatic compound having at least 9 carbon atoms to an aromatic product containing 6 - 8 carbon atoms comprising contacting the feedstock in the presence or absense of hydrogen with a zeolite catalyst having an X-ray diffraction pattern including the lines set out in Table I below:

Table I

| Interplanar d-Spacing (a) | Relative Intensity, I/Iox100 |
|---|---|
| 30.0 ± 2.2 | W-M |
| 22.1 ± 1.3 | W |

2. The process of claim 1 wherein the zeolite has an X-ray diffraction pattern as set out in Table II below:

Table II

| Interplanar d-Spacing (A) | Relative Intensity, I/Iox100 |
|---|---|
| 30.0 ± 2.2 | W-M |
| 22.1 ± 1.3 | W |
| 6.00 ± 0.1 | W-M |
| 4.06 ± 0.07 | W-S |
| 3.91 ± 0.07 | M-VS |

3. The process of claim 1 wherein the zeolite has an X-ray diffraction pattern as set out in Table III below:

Table III

| Interplanar d-Spacing (A) | Relative Intensity, I/Iox100 |
|---|---|
| 30.0 ± 2.2 | W-M |
| 22.1 ± 1.3 | W |
| 6.00 ± 0.1 | W-M |
| 4.06 ± 0.07 | W-S |
| 3.91 ± 0.07 | M-VS |

4. The process of claim 1 wherein the zeolite has an X-ray diffraction pattern as set out in Table IV below:

Table IV

| Interplanar d-Spacing (a) | Relative Intensity, I/Iox100 |
|---|---|
| 30.0 ± 2.2 | W-M |
| 22.1 ± 1.3 | W |
| 12.36 ± 0.2 | M-VS |
| 11.03 ± 0.2 | M-S |
| 8.83 ± 0.14 | M-VS |
| 6.86 ± 0.14 | W-M |
| 6.18 ± 0.12 | M-VS |
| 6.00 ± 0.10 | W-M |
| 5.54 ± 0.10 | W-M |
| 4.92 ± 0.09 | W |
| 4.64 ± 0.08 | W |

Table IV   (continued)

| Interplanar d-Spacing (a) | Relative Intensity, I/Iox100 |
|---|---|
| 4.41 ± 0.08 | W-M |
| 4.25 ± 0.08 | W |
| 4.10 ± 0.07 | W-S |
| 4.06 ± 0.07 | W-S |
| 3.91 ± 0.07 | M-VS |
| 3.75 ± 0.06 | W-M |
| 3.56 ± 0.06 | W-M |
| 3.42 ± 0.06 | VS |
| 3.30 ± 0.05 | W-M |
| 3.20 ± 0.05 | W-M |
| 3.14 ± 0.05 | W-M |
| 3.07 ± 0.05 | W |
| 2.99 ± 0.05 | W |
| 2.82 ± 0.05 | W |
| 2.78 ± 0.05 | W |
| 2.68 ± 0.05 | W |
| 2.59 ± 0.05 | W |

5. The process of any one of claims 3 to 6 wherein the zeolite has equilibrium adsorption capacities greater than 4.5 wt% for cyclohexane vapor and greater than 10 wt% for n-hexane vapor.

6. The process of any one of claims 3 to 6 wherein the zeolite has a composition comprising the molar relationship

$$X_2O_3:(n)YO_2,$$

wherein n is at least 10, X is aluminium and Y is silicon.

7. The process of any preceding claim wherein said at least one mononuclear aromatic compound is selected from trimethylbenzenes, dimethylbenzenes, diethylbenzenes and mixtures thereof.

8. The process of any preceding claim wherein said at least one mononuclear aromatic compound having at least 9 carbon atoms represents at least 3 wt% of the total feedstock, the balance of the feedstock comprising toluene and/or benzene.

9. The process of any preceding claim wherein said contacting is effected at a temperature of 90°C to 675°C, a pressure of 100 to 14,000 kPa, a hydrogen to hydrocarbon mole ratio of 0 to 10 and a weight hourly space velocity of 0.1 to 500.

**Patentansprüche**

1. Verfahren zur Umwandlung eines mindestens eine einkernige aromatische Verbindung mit mindestens 9 Kohlenstoffatomen enthaltenden Einsatzetoffs in ein 6 - 8 Kohlenstoffatome enthaltendes aromatisches Produkt, bei dem man den Einsatzstoff gegebenenfalls in Gegenwart von Wasserstoff mit einem Zeolithkatalysator, dessen Röntgenbeugungsmuster die in der nachfolgenden Tabelle I aufgeführten Linien enthält:

Tabelle I

| Interplanar d-Abstand (a) | Relative Intensität, I/Iox100 |
|---|---|
| 30,0 ± 2,2 | W-M |
| 22,1 ± 1,3 | W |

in Berührung bringt.

2. Verfahren nach Anspruch 1, bei dem man einen Zeolithen einsetzt, der ein Röntgenbeugungsmuster gemäß der nachfolgenden Tabelle II aufweist:

Tabelle II

| Interplanarer d-Abstand (A) | Relative Intensität, I/Iox100 |
|---|---|
| 30,0 ± 2,2 | W-M |
| 22,1 ± 1,3 | W |
| 6,00 ± 0,1 | W-M |
| 4,06 ± 0,07 | W-S |
| 3,9 ± 0,07 | M-SS. |

3. Verfahren nach Anspruch 1, bei dem man einen Zeolithen einsetzt, der ein Röntgenbeugungsmuster gemäß der nachfolgenden Tabelle III aufweist:

Tabelle III

| Interplanarer d-Abstand (A) | Relative Intensität, I/Iox100 |
|---|---|
| 30,0 ± 2,2 | W-M |
| 22,1 ± 1,3 | W |
| 6,00 ± 0,1 | W-M |
| 4,06 ± 0,07 | W-S |
| 3,9 ± 0,07 | M-SS. |

4. Verfahren nach Anspruch 1, bei dem man einen Zeolithen einsetzt, der ein Röntgenbeugungsmuster gemäß der nachfolgenden Tabelle IV aufweist:

## Tabelle IV

| Interplanarer d-Abstand (a) | Relative Intensität, I/Iox100 |
|---|---|
| 30,0 ± 2,2 | W-M |
| 22,1 ± 1,3 | W |
| 12,36 ± 0,2 | M-SS |
| 11,03 ± 0,2 | M-S |
| 8,83 ± 0,14 | M-SS |
| 6,86 ± 0,14 | W-M |
| 6,18 ± 0,12 | M-SS |
| 6,00 ± 0,10 | W-M |
| 5,54 ± 0,10 | W-M |
| 4,92 ± 0,09 | W |
| 4,64 ± 0,08 | W |
| 4,41 ± 0,08 | W-M |
| 4,25 ± 0,08 | W |
| 4,10 ± 0,07 | W-S |

| | | |
|---|---|---|
| 4,06 | ± 0,07 | **W-S** |
| 3,91 | ± 0,07 | **M-SS** |
| 3,75 | ± 0,06 | **W-M** |
| 3,56 | ± 0,06 | **W-M** |
| 3,42 | ± 0,06 | **SS** |
| 3,30 | ± 0,05 | **W-M** |
| 3,20 | ± 0,05 | **W-M** |
| 3,14 | ± 0,05 | **W-M** |
| 3,07 | ± 0,05 | **W** |
| 2,99 | ± 0,05 | **W** |
| 2,82 | ± 0,05 | **W** |
| 2,78 | ± 0,05 | **W** |
| 2,68 | ± 0,05 | **W** |
| 2,59 | ± 0,05 | **W** |

5. Verfahren nach einem der Ansprüche 3 bis 6, bei dem man einen Zeolithen mit einem Gleichgewichtsadsorptionsvermögen über 4,5 Gew.-% für Cyclohexandampf und über 10 Gew.-% für n-Hexandampf einsetzt.

6. Verfahren nach einem der Ansprüche 3 bis 6, bei dem man einen Zeolithen mit der als Molverhältnis ausgedrückten Zusammensetzung

$$X_2O_3 : (n)YO_2,$$

worin n mindestens 10 beträgt, X für Aluminium und Y für Silicium steht, einsetzt.

7. Verfahren nach einem der vorhergehenden Ansprüche, bei dem man die mindestens eine einkernige aromatische Verbindung unter Trimethylbenzolen, Dimethylbenzolen, Diethylbenzolen und deren Gemischen auswählt.

8. Verfahren nach einem der vorhergehenden Ansprüche, bei dem die mindestens eine einkernige aromatische Verbindung mit mindestens 9 Kohlenstoffatomen mindestens 3 Gew.-% des gesamten Einsatzstoffs ausmacht und der Rest des Einsatzstoffs aus Toluol und/oder Benzol besteht.

9. Verfahren nach einem der vorhergehenden Ansprüche, bei dem man das Inberührungbringen bei einer Temperatur von 90°C bis 675°C, einem Druck von 100 bis 14.000 kPa, einem Molverhältnis von Wasserstoff zu Kohlenwasserstoff von 0 bis 10 und einer Katalysatorbelastung von 0,1 bis 500 durchführt.

**Revendications**

1. Procédé de conversion d'une charge de départ contenant au moins un composé aromatique mononucléaire possédant au moins 9 atomes de carbone en un produit aromatique contenant de 6 à 8 atomes de carbone, comprenant la mise en contact de la charge de départ, en présence ou en l'absence d'hydrogène, avec un catalyseur à base de zéolite, possédant un réseau de diffraction des rayons X comprenant les raies présentées dans le tableau I qui suit :

Tableau I

| Espacement d interplanaire (a) | Intensité relative, I/Iox100 |
|---|---|
| 30,0 ± 2,2 | F-M |
| 22,1 ± 1,3 | F |

2. Procédé suivant la revendication 1, caractérisé en ce que la zéolite possède un réseau de diffraction des rayons X, tel que présenté dans le tableau II qui suit :

Tableau II

| Espacement d interplanaire (A) | Intensité relative, I/Iox100 |
|---|---|
| 30,0 ± 2,2 | F-M |
| 22,1 ± 1,3 | F |
| 6,00 ± 0,1 | F-M |
| 4,06 ± 0,07 | F-P |
| 3,91 ± 0,07 | F-TP |

3. Procédé suivant la revendication 1, caractérisé en ce que la zéolite possède un réseau de diffraction des rayons X, tel que présenté dans le tableau III qui suit :

Tableau III

| Espacement d interplanaire | Intensité relative, (A) I/Iox100 |
|---|---|
| 30,0 ± 2,2 | F-M |
| 22,1 ± 1,3 | F |
| 6,00 ± 0,1 | F-M |
| 4,06 ± 0,07 | F-P |
| 3,91 ± 0,07 | M-TP |

4. Procédé suivant la revendication 1, caractérisé en ce que la zéolite possède le réseau de diffraction des rayons X, tel que présenté dans le tableau IV qui suit :

## Tableau IV

| Espacement d interplanaire (a) | Intensité relative, I/Iox100 |
|---|---|
| 30,0 ± 2,2 | F-M |
| 22,1 ± 1,3 | F |
| 12,36 ± 0,2 | M-TP |
| 11,03 ± 0,2 | M-P |
| 8,83 ± 0,14 | M-TP |
| 6,86 ± 0,14 | F-M |
| 6,18 ± 0,12 | M-TP |
| 6,00 ± 0,10 | F-M |
| 5,54 ± 0,10 | F-M |
| 4,92 ± 0,09 | F |
| 4,64 ± 0,08 | F |
| 4,41 ± 0,08 | F-M |
| 4,25 ± 0,08 | F |
| 4,10 ± 0,07 | F-P |
| 4,06 ± 0,07 | F-P |
| 3,91 ± 0,07 | M-TP |
| 3,75 ± 0,06 | F-M |
| 3,56 ± 0,06 | F-M |
| 3,42 ± 0,06 | TP |
| 3,30 ± 0,05 | F-M |
| 3,20 ± 0,05 | F-M |
| 3,14 ± 0,05 | F-M |
| 3,07 ± 0,05 | F |
| 2,99 ± 0,05 | F |
| 2,82 ± 0,05 | F |
| 2,78 ± 0,05 | F |
| 2,68 ± 0,05 | F |
| 2,59 ± 0,05 | F |

5.  Procédé suivant l'une quelconque des revendications 3 à 6, caractérisé en ce que la zéolite possède un pouvoir d'adsorption à l'équilibre supérieur à 4,5% en poids pour la vapeur de cyclohexane et supérieur à 10% en poids pour la vapeur de n-hexane.

6.  Procédé suivant l'une quelconque des revendications 3 à 6, caractérisé en ce que la zéolite possède une composition telle qu'elle comprend la relation molaire

$$X_2O_3: (n)\ YO_2,$$

dans laquelle n est au moins égal à 10, X est l'aluminium et Y est le silicium.

7. Procédé suivant l'une quelconque des revendications précédentes, caractérisé en ce que ledit au moins un composé aromatique mononucléaire est choisi parmi les triméthylbenzènes, les diméthylbenzènes, les diéthylbenzènes et leurs mélanges.

8. Procédé suivant l'une quelconque des revendications précédentes, caractérisé en ce que ledit au moins un composé aromatique mononucléaire possédant au moins 9 atomes de carbone représente au moins 3% en poids de la charge de départ totale, le reste de la charge de départ étant constitué de toluène et/ou de benzène.

9. Procédé suivant l'une quelconque des revendications précédentes, caractérisé en ce que l'on procède à ladite mise en contact à une température de 90°C à 675°C, sous une pression de 100 à 14.000 kPa, un rapport molaire d'hydrogène à hydrocarbure de 0 à 10 et une vitesse spatiale horaire pondérale de 0,1 à 500.

FIG. 1

FIG. 2

FIG. 3

FIG. 4

FIG. 5

EP 0 457 853 B1